(19) Europäisches Patentamt
European Patent Office
Office européen des brevets

(11) Publication number : **0 447 168 A2**

(12) # EUROPEAN PATENT APPLICATION

(21) Application number : **91302035.0**

(22) Date of filing : **12.03.91**

(51) Int. Cl.$^5$ : **A61K 9/54**, A61K 9/52, A61K 9/16

(30) Priority : **16.03.90 JP 66190/90**
**30.11.90 JP 338919/90**

(43) Date of publication of application :
**18.09.91 Bulletin 91/38**

(84) Designated Contracting States :
**AT BE CH DE DK ES FR GB GR IT LI LU NL SE**

(71) Applicant : **YAMANOUCHI PHARMACEUTICAL CO. LTD.**
**No. 3-11 Nihonbashi-Honcho, 2-chome**
**Chuo-ku**
**Tokyo (JP)**

(72) Inventor : **Ohmura, Tadayoshi**
**2-3-11 Takayanagi**
**Fujieda-shi, Shizuoka, 426 (JP)**
Inventor : **Fukui, Mueneo**
**5-13-14 Minamisurugadai**
**Fujieda-shi, Shizuoka, 426 (JP)**
Inventor : **Hosono, Toshiharu**
**4-11-8-10A Yahata**
**Shizuoka-shi, Shizuoka, 422 (JP)**
Inventor : **Kajiyama, Atushi**
**5-20-4-2-208 Negishi**
**Urawa-shi, Saitama, 336 (JP)**
Inventor : **Mizumoto, Takao**
**4-13-28 Tanuma**
**Fujieda-shi, Shizuoka, 426 (JP)**

(74) Representative : **Geering, Keith Edwin et al**
**REDDIE & GROSE 16 Theobalds Road**
**London WC1X 8PL (GB)**

(54) **Long acting granular pharmaceutical composition.**

(57) A long acting granular composition which comprises (a) a fine particulate nucleus composed of a water-insoluble substance ; (b) a drug layer containing a drug which is difficult to prepare in long acting form and a non-swellable entero-soluble base ; and (c) a drug-release controlling film containing a drug-release controlling material and an entero-soluble base.

The long acting granular composition has a very satisfactory drug-release pattern and is free from the risk of abrupt disintegration in the digestive tract.

EP 0 447 168 A2

# LONG ACTING GRANULAR PHARMACEUTICAL COMPOSITION

The present invention relates to a long acting granular composition which is useful as a long acting pharmaceutical formulation for once-a-day administration.

Long acting formulations have many advantages in medical practice in that they help reduce the frequency of administration for improved patient compliance and enhanced therapeutic efficacy. For this reason, much ingenuity has been invested in dosage forms for such long acting formulations. However, it is never easy to develop a long acting formulation that would establish an effective drug concentration in blood soon after administration and yet provide a therapeutically constant drug concentration in blood over many hours following administration.

The inventors of the present invention previously succeeded in providing a semi-long acting preparation of nicardipine hydrochloride, one of the drugs known to be difficult to prepare in long acting form, by coating fine particulate nuclei such as Nonpareil with a coating composition containing nicardipine hydrochloride, an entero-soluble base and/or a gastro-soluble base and a surfactant, and coating the resulting granules further with a drug-release controlling film having drug-release controlling properties (see examined JP-1-7047). The above preparation has been used clinically as a semi-long acting granular composition for twice-a-day administration. However, from the viewpoint of therapeutic advantage, an oral preparation for once-a-day administration is keenly demanded.

With the thought that successful development of a pharmaceutical preparation for once-a-day administration primarily hinges on improvement of the drug-release controlling film, the inventors of the present invention developed a drugrelease controlling material which does not substantially release the drug in the stomach but releases it at a controlled rate under neutral conditions and at a rapid rate under alkaline conditions (pH 7.2 - 7.8). However, these granules which control the release of drug by the drug-release controlling film have the defects that they suffer gradual loss of strength with steady penetration of digestive juice and have the risk of disintegration which leads to an abrupt massive release of the drug.

As a result of extensive investigation to improve the above defects, the inventors of the present invention have succeeded in developing a long acting granular composition by utilizing selected types of materials for the particulate nuclei, the base used in the drug layer and the drug-release controlling film. The present invention provides a long acting granular composition comprising:

(a) a particulate nucleus composed of water-insoluble substance; around the nucleus

(b) a drug layer containing a drug which is difficult to prepare in long acting form and non-swellable entero-soluble base; and around the drug layer

(c) a drug-release controlling film containing drug-release controlling material and entero-soluble base.

The size of the nucleus (a) may be within a wide range and is not critical, suitably being of any size common for pharmaceutical granules or pellets; it can for example be up to 3mm, and is preferably at least several (e.g. 5 or 10)μ; often it will be from 100 to 1000 μ, e.g. 400 or 500 to 700 μ.

The granular composition of the present invention can have a good drug-release pattern and is free from the risk of abrupt disintegration in the digestive tract.

Suitable fine nucleus particles of water-insoluble substance to be used in accordance with the present invention include, among others, minute grairs obtainable by granulating crystalline cellulose, microcrystalline cellulose or the like into spherical form. A typical example of such microcrystalline cellulose is a commercial product available under the tradename Avicel PH101 (Asahi Chemical Industry Co., Ltd.). The non-swellable entero-soluble base mentioned above includes, among others, CMEC (tradename, Freund Sangyo K.K.: carboxymethylethylcellulose), hydroxypropylmethylcellulose phthalate, cellulose acetate phthalate, shellac and so on. The drug-release controlling material to be incorporated in the drug-release controlling film may be non-swellable or swellable.

The non-swellable drug-release controlling material includes, among others, ethylcellulose, acetylcellulose and so on, while the swellable material includes, among others, Eudragit RS (a tradename of Rohm and Haas Co.; a 1:2:0.1 copolymer of ethyl acrylate, methyl methacrylate and trimethyl-ammonioethyl methacrylate chloride) and Eudragit RS 100L (a tradename of Rohm and Haas Co.; a 1:2:0.2 copolymer of ethyl acrylate, methyl methacrylate and trimethylammonioethyl methacrylate chloride). Comonomer proportions herein are molar.

The entero-soluble base to be incorporated in the drug-release controlling film may also be non-swellable or swellable.

The former may include the same bases described as examples of the base incorporated in the drug layer. The latter base includes, among others, Eudragit L (a tradename of Rohm and Haas Co., a 1:1 copolymer of methyl methacrylate and methacrylic acid) and Eudragit S (a tradename of Rohm and Haas Co., a 2:1 copolymer of methyl methacrylate and methacrylic acid).

In accordance with the present invention, a long acting nicardipine granular composition, for example, can be manufactured by the following procedure. First, nicardipine or a salt thereof and the non-swellable entero-soluble base are dissolved in a solvent and the resulting solution is spray-coated on fine nucleus particles by such a technique as centrifugal fluidized coating method, fluidized-bed coating method or the like followed by frying to give granules. These granules are then spray-coated with a coating solution containing the drug-release controlling material, the entero-soluble base and a plasticizer in a solvent, followed by drying.

The solvent mentioned above includes, among others, methanol, ethanol, isopropyl alcohol, chloroform, acetone, methylene chloride, water and so on. While these solvents may be used independently, they can be used in appropriate combinations.

The drying mentioned above is preferably carried out at a low temperature conducive to effective removal of the solvent, for example 40°C, for a few hours.

In the course of manufacture under the above conditions, the nicardipine or salt thereof becomes amorphous.

In the practice of the present invention, pharmaceutical auxiliaries such as plasticizers, excipients, lubricants and/or binders can be used as needed in appropriate proportions. These additives are not particularly limited and those known and in common use can be employed.

The long acting granular composition thus manufactured can be administered alone as a pharmaceutical preparation for once-a-day administration, or in combination with a semi-long acting granular composition and/or an immediate drug-releasing granular composition. Such a combined pharmaceutical preparation may be a mixed granular composition obtainable by mixing the long acting granular composition described above with a semi-long acting granular composition and/or an immediate drug-releasing granular composition. Alternatively, the combined pharmaceutical composition may be a laminated granular composition obtainable by coating the long acting granular composition further with an immediate drug-releasing layer. Such a combined pharmaceutical preparation is advantageous in providing long acting preparations of a drug, such as nicardipine, which has only a very short half life in blood, is sparingly soluble under alkaline conditions and has a large first-pass effect. The semi-long acting granular composition (II) and/or immediate drug-releasing (gastro-soluble) granular composition (III) which can be used in combination with the long acting granular composition of the present invention (I) may for example be spherical granules as in above JP-1-7047. Thus, the semi-long acting granule (II) may be a spherical granule which comprises a fine particulate nucleus coated with a drug layer comprising (a) drug which is difficult to prepare in long acting form, such as amorphous nicardipine or a salt thereof and (b) one or more entero-soluble bases e.g. selected from hydroxypropylmethylcellulose phthalate, methyl methacrylate-methacrylic acid copolymer, cellulose acetate phthalate and shellac and/or one or more gastro-soluble bases e.g. selected from hydroxypropylcellulose, polyethylene glycol, methylcellulose and hydroxypropylmethylcellulose, and one or more surfactants e.g. selected from polyoxyethylene sorbitan monooleate, polyoxyethylene alkyl ether and polyoxyethylene hydrogenated castor oil; said drug layer may be coated with (c) one or more drug-release controlling materials e.g. selected from a copolymer of ethyl acrylate, methyl methacrylate and tri-methylammonioethyl methacrylate chloride, and ethylcellulose. The immediate drug-releasing granular composition (III) may be a spherical granule obtainable by coating a fine particulate nucleus with a coating composition containing drug which is difficult to prepare in long acting form and gastro-soluble base.

With combined pharmaceutical preparations such as the mixed granules and laminated granules, an effective drug concentration in blood can be promptly obtained after ingestion and a suiffciently high constant drug concentration can be maintained over a period of 24 hours. Therefore, these combination type compositions are advantageous for providing once-a-day preparations expected to exhibit immediate effects as well.

The present invention has been described taking nicardipine hydrochloride as a specific example of the drug which is difficult to prepare in long acting form. However, the present invention is very useful for other drugs, as well, which are generally acknowledged to be difficult to prepare in long acting form, e.g. which fall within one or more of the following categories :

(1) Drugs with short elimination half lives ($t_{1/2}$) in the blood.

(2) Drugs with varying absorption rates according to the sites in the gastrointestinal tract.

(3) Drugs which are sparingly soluble at high pH values.

(4) Drugs which are subject to a marked first-pass effect in the liver.

Among drugs having such properties are amosulalol, nicardipine, propranolol, diltiazem, nifedipine, isosorbide dinitrate, cephalexin and so on.

As the following disintegration test example indicates, the long acting granular composition of the present invention retains sufficient strengh in phosphate buffer for many hours. The composition also shows a satisfactory drug concentration pattern in blood as evidenced in animal experiments using beagle dogs.

TEST EXAMPLE 1 (DISINTEGRATION TEST)

The granules prepared in Comparative Example 1, Example 1 and Example 2 were respectively stirred in phosphate buffer (pH 7.2) at 37°C and sampled for evaluation of grain strength at time intervals. The measured grain strength was expressed in units of the load per grain which caused disintegration of the granules.

In the drug-release controlling film of the granule according to comparative Example 1, liquefaction due to dissolution of the nucleus began to occur within 30 minutes after the beginning of the test, indicating a marked decrease in grain strength with time. On the other hand, the granules of Examples 1 and 2 showed decreases in strength with time but still retained fairly high strength even after 4 hours. The results of the test are shown in Table 1.

TABLE 1

| Time (hours) | 0 | 0.5 | 1 | 2 | 4 |
|---|---|---|---|---|---|
| Strength of the granule of Comparative Example 1 (g) | 778 | 55 | 12-18 | 9 | 7 |
| Strength of the granule of Example 1 (g) | 1270 | 920 | 860 | 200 | 110 |
| Strength of the granule of Example 2 (g) | 1290 | 880 | 445 | 300 | 250 |

It will be apparent from the above table that the granule according to the present invention is considerably higher in strength than the granule of Comparative Example 1, viz. 16-fold as high in the case of the granule of Example 1 and 36-fold as high in the case of the granule of Example 2.

TEST EXAMPLE 2

The capsules each containing 240 mg of nicardipine hydrochloride as prepared in accordance with Comparative Example 2 and Example 3 were respectively administered, together with 20 ml of water, orally to male beagle dogs in groups of 5 once a day for 4 consecutive days for comparison.

The blood was sampled at time intervals after administration and the drug concentration in plasma was determined. The mean drug concentrations in plasma on the 4th day are shown in Fig. 1. Whereas the composition of Comparative Example 2 showed a sharp increase in plasma concentration, the composition of Example 3 showed an excellent drug concentration pattern as a long acting preparation.

TEST EXAMPLE 3

The capsule containing 90 mg of nicardipine hydrochloride prepared in Example 5 was orally administered to seven healthy volunteers once a day postprandially for 4 consecutive days and the drug concentrations in plasma on the 1st and 4th days were determined. The results are shown in Fig. 2.

EXAMPLES

The Examples given below are intended to illustrate the present invention in further detail and should by no means be construed as defining the metes and bounds of the invention. In Reference Examples, processes for preparation of an immediate drug-releasing granular composition and an entero-soluble granular composition (a semi-long acting preparation) are described. In Comparative Examples, a granular composition prepared using Nonpareil (tradename, Freund Sangyo Co., Ltd.) as a water-soluble nucleus is described.

## REFERENCE EXAMPLE 1

Using 2.6 kg of a coating solution prepared by dissolving 200 g of nicardipine hydrochloride, 275 g of hydroxypropylmethylcellulose, 25 g of Macrogol 6000 and 20 g of Macrogol 400 in a 1:1 (w/w) mixture of methanol and methylene chloride, 500 g of Nonpareil 105 was coated by the fluidized-bed coating method and dried to provide an immediate drug-releasing granular composition.

## REFERENCE EXAMPLE 2

Using 3.0 kg of a homogeneous solution prepared by dissolving 200 g of nicardipine hydrochloride, 200 g of Eudragit L 100 and 50 g of tween 80 in a 1:1 (w/w) mixture of methanol and methylene chloride, 500 g of Nonpareil 103 was coated by the fluidized-bed coating method and dried to provide granules. A 500 g portion of thus obtained granules was further coated with 200 kg of a coating solution prepared by dissolving 18.2 g of Eudragit RS 100 L and 1.8 a of Macrogol 400 in a 1:1 (w/w) mixture of methanol and methylene chloride and dried to provide entero-soluble granules.

## COMPARATIVE EXAMPLE 1

Using 3.0 kg of a homogenous solution prepared by dissolving 455 g of nicardipine hydrochloride, 227 g of Eudragit L 100 and 68 g of Tween 80 in methanol-methylene chloride (1:1, w/w), 500 g of Nonpareil 103 was coated by the fluidized-bed coating method and dried to provide granules. A 400 g portion of the dried granules was further coated with 110 g of a coating solution prepared by dissolving 9.0 g of Eudragit RS 100, 1.0 g of Eudragit L 100 and 0.1 g of Macrogol 400 in methanol-methylene chloride (1:1, w/w) and dried. A 400 g portion of the resulting granules was further coated with an aqueous solution containing 400 g of Eudragit 30 D55 and 12 g of triethyl citrate in 400 g of water and dried to provide an entero-soluble granular composition.

## COMPARATIVE EXAMPLE 2

The granular compositions prepared in Comparative Example 1, Reference Example 1 and Reference Example 2 were mixed in a ratio of 8:1:3 in terms of the drug content and filled into hard capsules to provide nicardipine hydrochloride capsules.

## EXAMPLE 1

Using a stir granulating machine, 3.5 kg of Avicel PH101 was granulated with 3.85 kg of water to provide nuclei which were then dried. A 500 g portion of the dried nuclei was then coated with 5.25 kg of a homogeneous solution prepared by dissolving 341 g of nicardipine hydrochloride, 341 g of carboxymethylethylcellulose and 68 g of Tween 80 in methanol-methylene chloride (1:1, w/w) by the fluidized-bed coating method and dried to give drug-containing granules. A 400 g portion of these dry granules was further coated with 660 g of a solution prepared by dissolving 45 g of ethylcellulose, 15 g of carboxymethylethylcellulose and 6 g of triethyl citrate in methanol-methylene chloride (1:1, w/w) to provide granules each having a drug-release controlling film. These granules were dried at 40°C for 4 hours and filled into capsules in conventional manner to provide nicardipine hydrochloride capsules.

## EXAMPLE 2

Using a stir granulating machine, 3.5 kg of Avicel PH101 was granulated with 3.85 kg of water and the resulting nuclei were dried. A 500 g portion of the dry nuclei was coated with 6.25 kg of a homogenous solution prepared by dissolving 341 g of nicardipine hydrochloride, 341 g of carboxymethylethylcellulose and 68 g of Tween 80 in methanol-methylene chloride (1:1, w/w) by the fluidized-bed coating method and dried to prepare drug-containing granules. A 400 g portion of these granules was further coated with 200 g of a solution prepared by dissolving 16.4 g of Eudragit RS100, 1.8 g of Eudragit L 100 and 1.8 g of triethyl citrate in methanol by the fluidized-bed coating method to provide granules having a drug-release controlling film. The granules were dried at 40°C for 4 hours and filled into capsules in conventional manner to provide nicardipine hydrochloride capsules.

EXAMPLE 3

The granules prepared in Example 1 and Reference Example 1 were mixed in a ratio of 11:1 in terms of the drug content and filled into hard capsules to provide nicardipine hydrochloride capsules.

EXAMPLE 4

The granules prepared in Example 1 and Reference Example 1 were mixed in a ratio of 7:1 in terms of the drug content and filled into hard capsules to provide nicardipine hydrochloride capsules.

EXAMPLE 5

The granules prepared in Example 2 and Reference Example 1 were blended in a ratio of 8:1 in terms of the drug content and filled into hard capsules to provide nicardipine hydrochloride capsules.

EXAMPLE 6

Using a stir granulating machine, 3.5 kg of Avicel PH101 was granulated with 3.85 kg of water and dried to give nuclei. A 400 g portion of the dry nuclei was coated with a homogeneous solution prepared by dissolving 505 g of nicardipine hydrochloride, 404 g of carboxymethylethylcellulose and 91 g of Tween 80 by the fluidized-bed coating method and dried to prepare drug-containing granules. A 400 g portion of these granules was further coated with 213 g of a solution prepared by dissolving 13.4 g of Eudragit RS100, 1.5 g of Eudragit L 100 and 1.1 g of triethyl citrate in methanol by the fluidized-bed coating method to prepare granules each having a drug-release controlling film. The granules were then dried at 40°C and further coated with 231 g of a homogeneous solution prepared by dissolving 17.3 g of nicardipine hydrochloride and 17.3 g of hydroxypropylcelluose in ethanol by the fluidized-bed coating method and dried to give long-acting granules. The granules were then filled into capsules in conventional manner to provide nicardipine hydrochloride capsules.

EXAMPLE 7

The long acting granules prepared in Example 6 (260 g) were mixed homogeneously with 170 g of crystalline cellulose, 30 g of polyvinylpyrrolidone (tradename KOLLIDON VA64, made by BASF Co.) and 2.3 g of magnesium stearate and the mixture was compressed into tablets each weighing 462 mg.

While the invention has been described in detail and reference with specific embodiments thereof, it will be apparent to one skilled in the art that various changes and modifications can be made therein without departing therefrom.

## Claims

1. A long acting pharmaceutical composition comprising granules which have
   (a) a particulate nucleus composed of water-insoluble substance; around the nucleus
   (b) a drug layer containing drug which is difficult to prepare in long acting form and non-swellable entero-soluble base; and around the drug layer
   (c) a drug-release controlling film containing drug-release controlling material and entero-soluble base.

2. A composition according to claim 1 wherein said nucleus comprises microcrystalline cellulose, said non-swellable entero-soluble base of said drug layer comprises carboxymethylethylcellulose, said drug-release controlling material comprises ethylcellulose, and said entero-soluble base of said drug-release controlling film comprises carboxymethylethylcellulose.

3. A composition according to claim 1 wherein said nucleus comprises microcrystalline cellulose, said non-swellable entero-soluble base of said drug comprises carboxy-methylethylcellulose, said drug-release controlling material comprises a 1:2:0.2 (molar) copolymer of ethyl acrylate, methyl methacrylate and trimethylammonioethyl methacrylate chloride, and said entero-soluble base of said drug-release controlling film comprises a 1:1 (molar) copolymer of methyl methacrylate and methacrylic acid.

4. A composition according to claim 1, 2 or 3 wherein said drug comprises nicardipine or salt thereof.

5. A composition according to any preceding claim in combination with
   1) a semi-long acting granular drug composition and/or
   2) an immediate drug-releasing granular pharmaceutical composition.

6. A composition according to any of claims 1 to 4 wherein layer (c) is coated with (d) an immediate drug-releasing layer containing drug which is difficult to prepare in long acting form - e.g. nicardipine or salt thereof.

7. A composition according to claim 5 wherein the drug in composition (1) and/or composition (2) comprises nicardipine or salt thereof.

Fig. 1

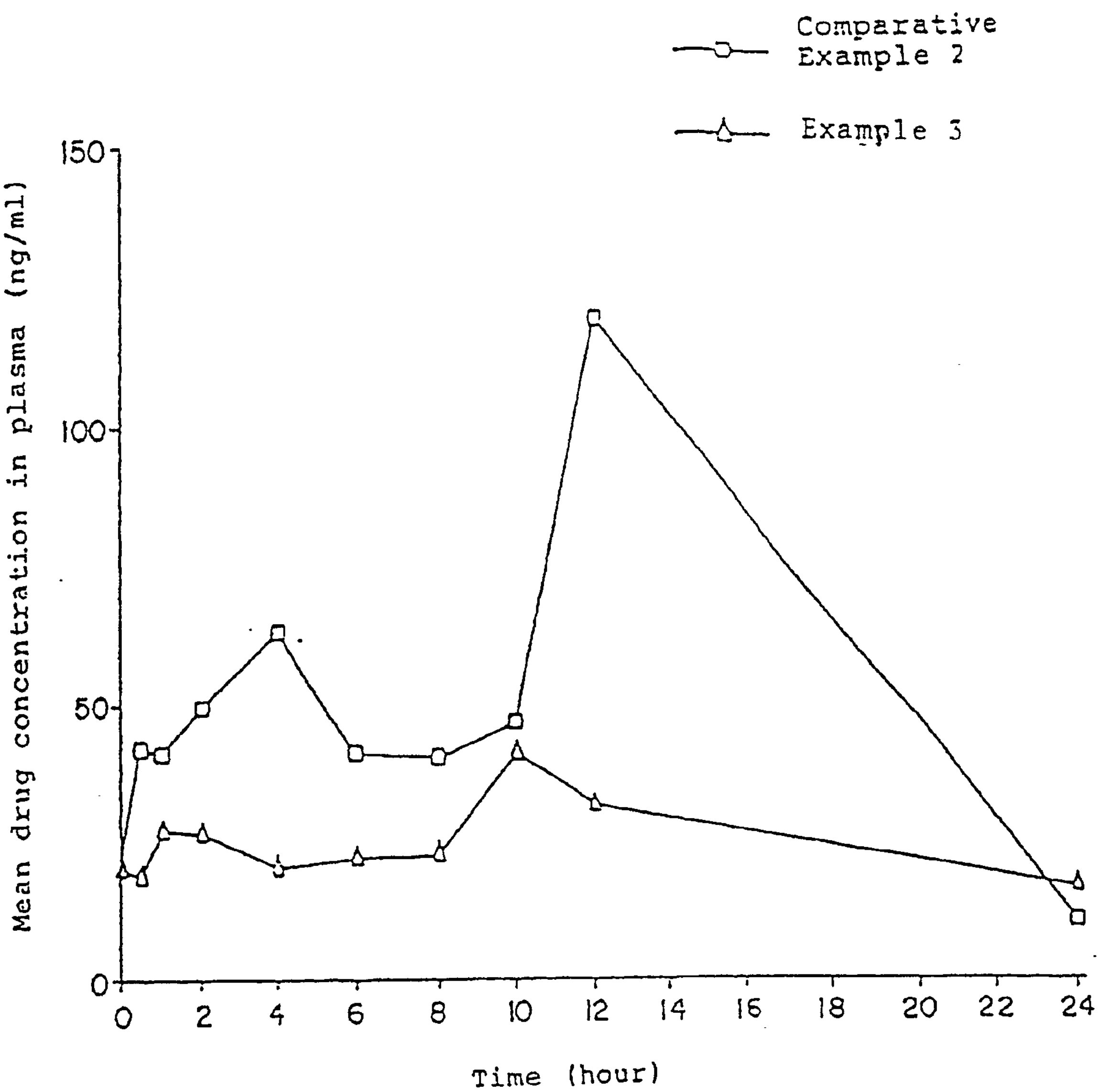
Comparative Example 2
Example 3
150
100
50
0
Mean drug concentration in plasma (ng/ml)
0
2
4
6
8
10
12
14
16
18
20
22
24
Time (hour)

Fig. 2

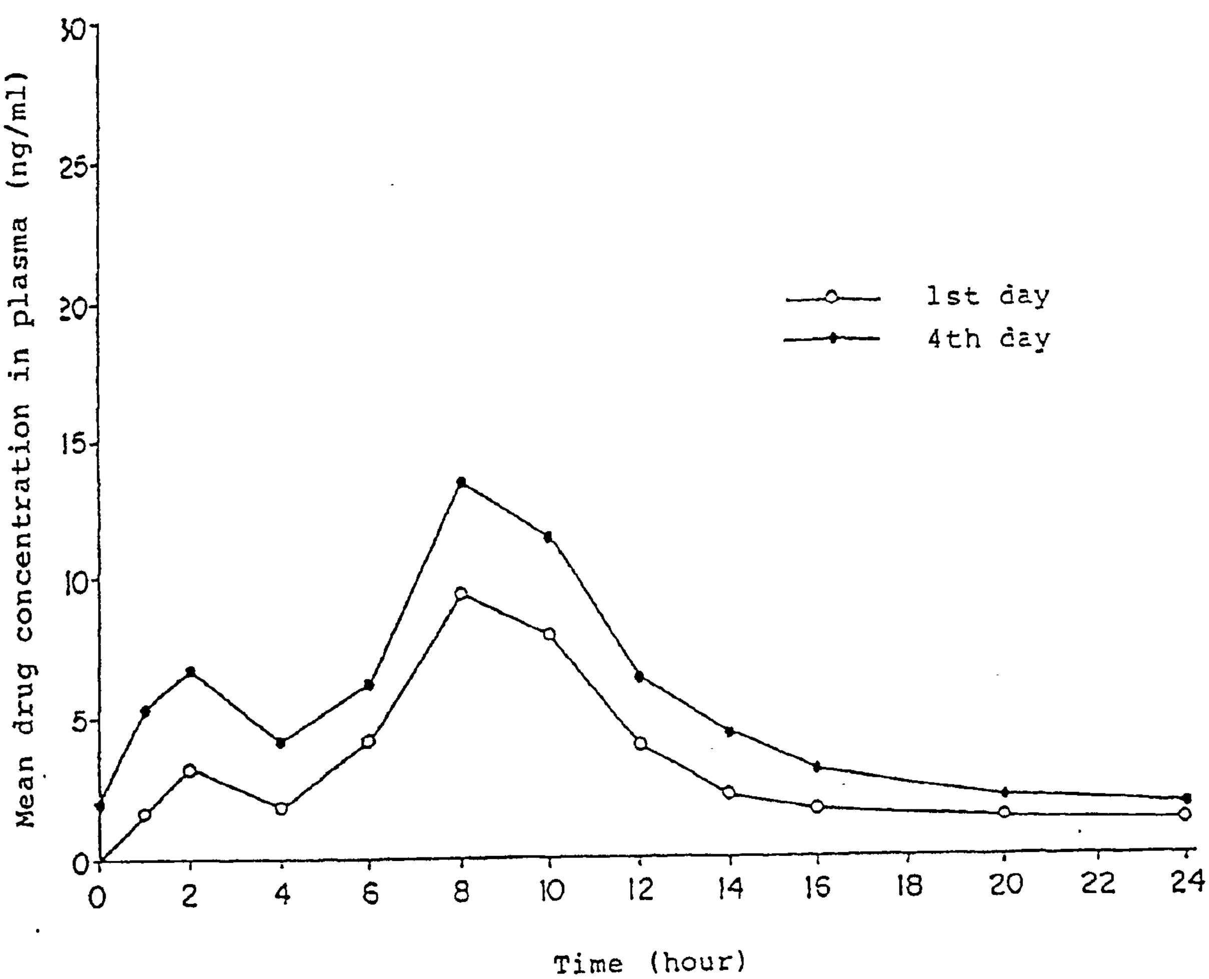
30
25
20
15
10
5
0
Mean drug concentration in plasma (ng/ml)
1st day
4th day
0
2
4
6
8
10
12
14
16
18
20
22
24
Time (hour)